(19) Europäisches Patentamt
European Patent Office
Office européen des brevets

(11) Veröffentlichungsnummer: **0 287 068 B1**

(12) **EUROPÄISCHE PATENTSCHRIFT**

(45) Veröffentlichungstag der Patentschrift: **13.11.91**

(51) Int. Cl.⁵: **A61M 16/01**

(21) Anmeldenummer: **88105886.1**

(22) Anmeldetag: **13.04.88**

(54) **Inhalations-Anästhesiegerät.**

(30) Priorität: **14.04.87 DE 3712598**

(43) Veröffentlichungstag der Anmeldung:
**19.10.88 Patentblatt 88/42**

(45) Bekanntmachung des Hinweises auf die
Patenterteilung:
**13.11.91 Patentblatt 91/46**

(84) Benannte Vertragsstaaten:
**CH DE ES FR GB IT LI NL SE**

(56) Entgegenhaltungen:
**DE-C- 576 139**
**FR-A- 1 043 855**
**FR-A- 2 325 392**
**US-A- 3 283 754**
**US-A- 3 592 191**

(73) Patentinhaber: **Siemens Elema AB**
**Röntgenvägen 2**
**S-171 95 Solna 1(SE)**

(84) Benannte Vertragsstaaten:
**SE**

Patentinhaber: **SIEMENS AKTIENGESELL-
SCHAFT**
**Wittelsbacherplatz 2**
**W-8000 München 2(DE)**

(84) Benannte Vertragsstaaten:
**CH DE ES FR GB IT LI NL**

(72) Erfinder: **Olsson, Sven Gunnar**
**Villa Fortuna**
**S-232 00 Arloev(SE)**
Erfinder: **Rydgren, Göran**
**Folksangsgatan 197**
**S-214 79 Malmö(SE)**

**Beschreibung**

Die Erfindung betrifft ein Inhalations-Anästhesiegerät gemäss dem Oberbegriff des Patentanspruches 1. Ein Inhalations-Anästhesiegerät mit einem geschlossenen Kreislauf ist beispielsweise aus der EP 0 121 255 A2 bekannt. Zur Reduzierung des Verbrauches an Anästhetikum wird hierbei das ausgeatmete Gas über einen $CO_2$-Absorber wieder der zum Patienten führenden Inspirationsleitung zugeführt. Über zusätzliche Leitungen kann verbrauchter Sauerstoff und Narkosegas hinzugefügt werden. Zur Überwachung sind an die Exspirationsleitung ein $CO_2$-Analysator und ein $O_2$-Analysator angeschlossen.

Ein ähnliches Narkose-Beatmungssystem ist aus der DE 29 45 472 C2 bekannt. Hier wird das Ausatmungsgas in einem Balg gesammelt und mit Narkose-Frischgas gemischt. Bei der nächsten Inspirationsphase wird das Gasgemisch aus diesem Balg über einen $CO_2$-Absorber zum Patienten geleitet. Der Überschuss an Narkose-Frischgas wird über ein Ventil in die Umgebung abgelassen.

Aus der DE 29 42 623 A1 ist ein Inhalations-Anästhesiegerät mit geschlossenem Kreislauf bekannt, bei dem kontinuierlich Narkose-Frischgas dem Kreislauf zugeführt wird und im Gegensatz zum aus der DE 29 45 472 C2 bekannten Gerät das Überschussgas in einem Gasevakuator aufgefangen wird. Von diesem Gasevakuator kann das Gas an einen Abscheider weitergeleitet werden, in dem das Anästhetikum wiedergewonnen werden kann. Durch das Auffangen des Überschussgases wird hierbei bereits eine Belastung der Umgebung mit Narkosegas vermieden.

Aus der US-PS 3,741,208 ist ein Lungenventilator bekannt, der für ein Inhalations-Anästhesiegerät mit einem geschlossenen Kreislauf verwendet werden kann. Für weitere Einzelheiten eines Lungenventilators im Zusammenhang mit dem erfindungsgemässen Inhalations-Anästhesiegerät wird auf diese Schrift verwiesen. Weiterhin ist daraus bekannt, dass bei einem geschlossenen System das Ausatmungsgas über einen Kompressor zur Inspirationsleitung zurückgeführt wird, nachdem unerwünschte Gaskomponenten, wie Kohlendioxyd, herausgefiltert wurden und verbrauchte Gaskomponenten, wie Sauerstoff, zugegeben wurden.

Alle bekannten geschlossenen Inhalations-Anästhesiegeräte haben gemeinsam, dass über eine Inspirationsleitung Narkosegas, d.h. eine Mischung aus Anästhetikum und Sauerstoff, dem Patienten zugeführt wird und dass das Ausatmungsgas über die Exspirationsleitung und Filter direkt wieder der Inspirationsleitung zugeführt wird. Zusätzlich wird jeweils verbrauchtes Gas ersetzt und möglicherweise Überschussgas an die Umgebung oder eine Wiedergewinnungsanlage abgeführt. Bei keinem der bekannten Systeme ist die Zusammensetzung des Gasgemisches in der Inspirationsleitung sicher festgelegt. Mit Ausnahme des aus der EP 0 121 255 A2 bekannten Systems arbeiten die anderen Systeme alle mit einem Überschuss an Narkose-Frischgas. Eine Kontrolle über den Narkosegasverbrauch existiert bei diesen Systemen nicht. Bei dem Inhalations-Anästhesiegerät gem. der EP 0 121 255 A2 wird zwar mit Hilfe eines Spirometers der Volumenverlust während eines Atemzuges bestimmt und daraus und der gemessenen Sauerstoffkonzentration der Narkosegasverbrauch ermittelt, aber auch hier ist die Zusammensetzung des Gases in der Inspirationsleitung unsicher. Bei allen Systemen ist der $CO_2$-Absorber in der Inspirationsleitung oder unmittelbar davor angeordnet. Die Funktion dieses Absorbers wird überhaupt nicht kontrolliert, so daß bei einer Sättigung oder dem Ausfall dieses Absorbers ungereinigtes Gas zum Patienten zurückgeführt würde.

Aus der US-A-3,592,191 ist ein halbgeschlossenes Anästhesiegerät bekannt, bei dem mit hohem Überschuß von Narkose-Frischgas gearbeitet wird. Das Ausatmungsgas wird über einen gekühlten Kondensator geleitet, in dem das Narkosegas kondensiert und von dem aus es über eine Leitung dem Narkosemittelvergaser zugeführt wird, von dem es erneut als Narkose-Frischgas zur Inhalationsleitung gelangt. Bei diesem halbgeschlossenen System mit hohem Überschuß an Narkose-Frischgas muß eine sehr große Menge von Gas aufbereitet werden. Dazu sind aufwendige Kühltechniken notwendig, möglichst sogar in zwei Stufen, wobei in der ersten Stufe die Feuchtigkeit aus dem Ausatmungsgas kondensiert und abgeleitet wird und erst in der zweiten Kühlstufe das Narkosegas kondensiert wird. Da in diesen Kondensatoren das Narkosegas nicht zu 100 % kondensiert, geht immer noch ein erheblicher Anteil an Narkosegas verloren. Insbesondere bei teuren Narkosegasen ist das schon aus Kostengründen unerwünscht. Weiterhin hat dieses halbgeschlossene System den Nachteil, daß beim Versagen des $CO_2$ Filters das ungereinigte Ausatmungsgas direkt zur Inspirationsleitung zurückgeführt wird.

Der vorliegenden Erfindung liegt die Aufgabe zugrunde, bei einem Inhalations-Anästhesiegerät der eingangs genannten Art, bei dem mit einem minimalen Anästhetikum Verbrauch gearbeitet werden kann, so zu verbessern, daß mit geringerer Narkose-Frischgaszufuhr zum Patienten und ohne aufwendige Kühlverfahren der Verbrauch an Anästhetikum minimal ist und trotzdem sichergestellt ist, daß der Patient quasi mit Narkose-Frischgas definierter Zusammensetzung beatmet wird. Eine weitere Aufgabe der Erfindung ist es, sicherzustellen, daß auch beim vorübergehenden Versagen der Filter oder der Rückführung des ausgeatmeten Gases die Anästhesie ohne Unterbrechung fortgestzt werden kann.

Diese Aufgaben werden erfindungsgemäss durch die Merkmale des Anspruches 1 gelöst. Gemäß der Erfindung ist ein Reservoir - zumindest für das Anästhetikum - vorgesehen. Von diesem Reservoir wird das Anästhetikum mit Hilfe eines Ventilators den Lungen eines Patienten zugeführt, wobei andere Gase, wie Sauerstoff, beigemischt werden können. Als Ventilator kann ein wie in der US-PS 3,741,208 beschriebenes Gerät verwendet werden. Die Erfindung ist jedoch unabhängig von der Art des verwendeten Beatmungsgerätes. Wesentlich ist lediglich, daß mit Hilfe eines derartigen Gerätes das gewünschte Gasgemisch den Lungen eines Patienten zugeführt und von diesen weggeleitet wird. Das Ausatmungsgas wird gem. der Erfindung über mindestens ein Filter, in dem vorbestimmte Gaskomponenten, wie Kohlendioxyd oder aber auch Wasser, herausgefiltert werden, gasförmig zu dem Reservoir zurückgeführt. Bei jedem neuen Atemzug wird damit dem Patienten mit einer definierten Zusammensetzung Narkose-Frischgas aus dem Reservoir zugeführt. Das Reservoir ist dabei so groß gwählt, daß mindestens die für die Narkose eines Patienten benötigte Menge an Anästhetikum darin enthalten ist. Nach der Reinigung des Ausatmungsgases durch die Filter liegt praktisch reines Narkose-Frischgas vor, das wieder zu dem Reservoir zurückgeführt ist.

Wenn das Reservoir zu Beginn der Anästhesie reines Anästhetikum enthält, wird diesem-in einem Mischer beispielsweise oder auch direkt in der Inspirationsleitung-Sauerstoff beigemischt.

Mit Hilfe des erfindungsgemäßen Inhalations-Anästhesiegerätes lassen sich die Verluste an Anästhetikum soweit begrenzen, daß praktisch nur das im Patienten metabolisierte oder durch die Haut diffundierte Gas ersetzt werden muß. Neben den Verlusten ist auch noch die Zufuhr an frischem Anästhesiegas auf ein Minimum beschränkt, d.h., hier kann im Gegensatz zu bekannten Systemen ohne Überschuß an Anästhetikum gearbeitet werden. Erst dadurch wird es kostenmässig möglich, auch extrem teure Anästhesiegase, wie Xenon, zu verwenden.

Ein weiterer Vorteil besteht darin, daß - unabhängig von dem verwendeten Anästhetikum - auf eine Absaugung von Überschußgas zu einem großen Sammelbehälter außerhalb des Behandlungsraumes verzichtet werden kann.

Ohne Schwierigkeiten lassen sich auch schnell unterschiedliche Volumina an zugeführtem Narkosefrischgas einstellen. Selbst vorübergehend im Körper des Patienten gelöste Narkosegase können - soweit sie später ausgeatmet werden - damit wieder dem System zugeführt werden und gehen nicht verloren. Die dem Reservoir zurückgeführten Narkosegase können anschliessend wieder verwendet werden.

In einer vorteilhaften Weiterbildung der Erfindung ist vorgesehen, dass das Reservoir ein Gasgemisch aus einem Anästhetikum und einem anderen Gas, vorzugsweise Sauerstoff, enthält. In diesem Fall kann direkt dieses Gasgemisch über den Ventilator den Atemwegen zugeführt werden

Der grosse Vorteil dieses erfindungsgemässen Inhalations-Anästhesiegerätes besteht darin, dass der Verbrauch an Anästhetikum auf ein absolutes Minimum eingeschränkt wird und das im Ausatmungsgas enthaltene Anästhetikum wieder direkt in das Reservoir zurückgeführt wird. Selbst wenn die Filter mangelhaft funktionieren sollten, lässt sich die Narkose unbehindert fortsetzen, während Gegenmassnahmen zum Beseitigen des mögliches Fehlers ergriffen werden. Selbst wenn ein Teil schlecht gereinigtes Ausatmungsgas zum Reservoir zurückströmen sollte, wird das in dem Gesamtvolumen so stark verdünnt, dass für eine längere Zeit das dem Patienten zugeführte Narkosegas in der Zusammensetzung kaum verändert wird.

In einer vorteilhaften Weiterbildung der Erfindung ist hierfür vorgesehen, dass zumindest zwischen Filter und Reservoir ein Gasanalysator vorgesehen ist, der die Zusammensetzung des Gases in dieser Leitung überwacht und beim ersten Anzeichen eines Fehlers in der Gaszusammensetzung zumindest einen Alarm auslöst. In einer vorteilhaften Weiterbildung kann das Ausgangssignal dieses Gasanalysators direkt dazu verwendet werden, die Rückfuhr des Gases zum Reservoir zu unterbinden und dieses beispielsweise in eine Auffangflasche zu leiten.

In einer vorteilhaften Weiterbildung der Erfindung ist vorgesehen, dass mindestens zwei Reservoire mit unterschiedlichen Gasmischungen und/oder mit unterschiedlichen Anästhetikumkonzentrationen vorgesehen sind. In diesem Fall ist in die Leitung zwischen Filter und diesen beiden Reservoiren ein Umschaltventil eingesetzt, das in Abhängigkeit von der mit dem Gasanalysator ermittelten Gaszusammensetzung und/oder Konzentration die Leitung mit dem entsprechenden Reservoir verbindet. Beispielsweise kann es sich hier um Reservoir handeln, von denen eins eine sehr hohe Konzentration an Anästhetikum enthält, wie es oft für den Beginn einer Narkose wünschenswert ist und das andere eine geringere Anästhetikumkonzentration und dafür eine höhere Sauerstoffkonzentration, wie es für eine längerwährende Narkose ausreicht. Ebenso ist es natürlich möglich, unterschiedliche Anästhetika in den verschiedenen Rerservoiren zu haben.

In einer vorteilhaften Weiterbildung der Erfindung ist vorgesehen, dass als Reservoir mindestens eine Druckflasche vorgesehen ist und dass in der Leitung zwischen Filter und Reservoir ein Kompressor vorgesehen ist. Die Anwendung dieses erfindungsgemässen Inhalations-Anästhesiegerätes ist besonders bei der Verwendung von Xenon als Anästhetikum vorteilhaft. Xenon ist ein sehr teueres Edelgas und in der Narkosewirkung den bisher geläufigen Narkosegasen, wie Halothan oder Lachgas darin überlegen, dass es keine Nebenwirkungen aufzeigt und sehr viel schneller vollständig wieder aus dem Körper entweicht, so dass die Narkosewirkung nach Beendigung der Anästhesie gezielt sehr viel schneller beendet werden kann.

In einer vorteilhaften Weiterbildung der Erfindung kann es vorgesehen sein, dass beispielsweise die Filter in bekannter Weise doppelt ausgeführt sind, wobei jeweils ein Filtersatz in die Leitung eingeschaltet ist und der andere in der Zwischenzeit gereinigt werden kann.

Weitere Einzelheiten und Vorteile der Erfindung ergeben sich aus den Unteransprüchen und im Zusammenhang mit der Beschreibung des Ausführungsbeispieles, das im folgenden anhand einer Figur beschrieben wird.

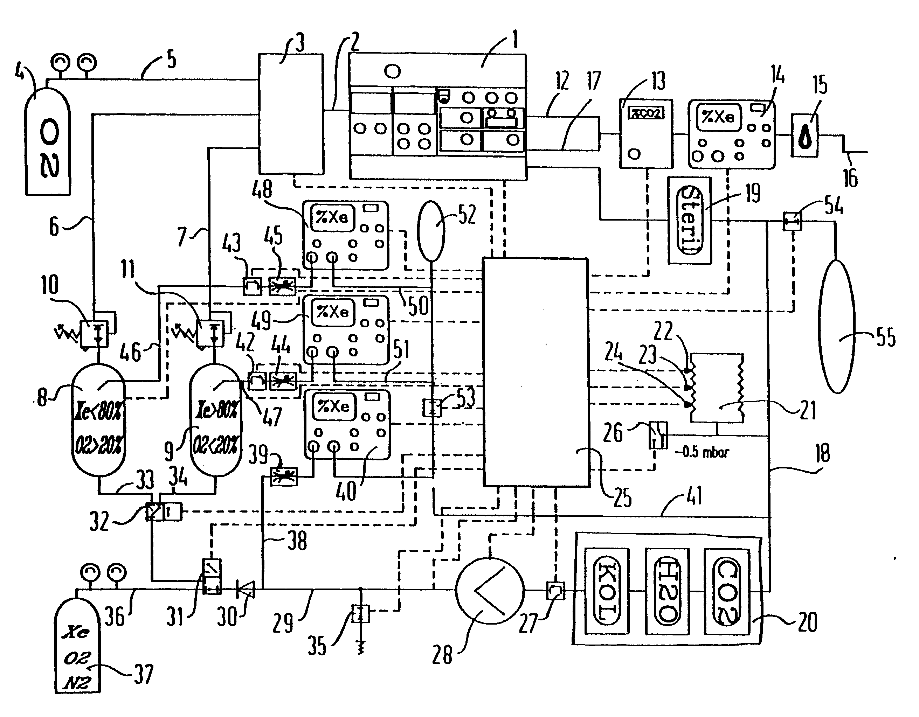

Die einzige Figur zeigt dabei schematisch ein erfindungsgemässes Inhalations-Anästhesiegerät.

Die Figur zeigt einen Ventilator (1), beispielsweise einen Servo-Ventilator, wie er aus dem Operating manual für den Servo-Ventilator 900 C der Firma Siemens-Elema AB bekannt ist. Diesem Ventilator wird über eine Leitung (2) Gas von einem Mischer (3) zugeführt. An diesen Mischer sind in diesem Ausführungsbeispiel eine Sauerstoff-Druckflasche (4) über eine Leitung (5) sowie über Leitungen (6 und 7) zwei Druckflaschen (8 und 9) angeschlossen, wovon die Druckflasche (8) ein Gasgemisch mit einer Konzentration von Xenon < 80 % und einer Sauerstoffkonzentration > 20 % enthält und die Flasche (9) eine Xenon-Konzentration > 80% und Sauerstoffkonzentration < 20%. Zwischen den als Reservoir dienenden Druckflaschen (8 und 9) und den Mischer (3) sind in die Leitungen (6 und 7) zwei Druckreduzierventile (10) bzw. (11) eingeschaltet. Vom Ventilator (1) wird das Gas über eine Inspirationsleitung (12), zwei Gasanalysatoren (13 bzw. 14) sowie einen Befeuchter (15) und eine Leitung (16) einem Patienten zugeführt. Das Ausatmungsgas wird wieder über die Gasanalysatoren und eine Exspirationsleitung (17) zum Ventilator (1) zurückgeführt und gelangt von diesem über eine Leitung (18) und möglicherweise ein Sterilfilter (19) zu einem Filter (20). Bei diesem Filter kann es sich um Absorbtions- oder Adsorbtions-Filter handeln und im vorliegenden Beispiel enthält dieses Filter einen speziellen Block für die Absorbtion von Kohlendioxyd, einen weiteren für die Absorbtion von Wasser sowie ein Kohlefilter für die Absorbtion von beispielsweise Halothan. An die Leitung (18) ist ein Nulldruckbehälter (21), in diesem Fall ein Faltenbalg, angeschlossen. Dieser Faltenbalg ist mit drei elektronischen Lagesensoren (22 - 24) ausgerüstet, deren Signale auf eine Steuerelektronik (25) gegeben sind, die später noch näher erläutert wird. Weiterhin ist an die Leitung (18) ein Drucksensor (26) angeschlossen, der beim Auftreten eines Unterdruckes von in diesem Beispiel grösser als -0,5 mbar ein Signal an die Steuerelektronik (25) abgibt. Auf den Drucksensor kann unter Umständen verzichtet werden. Beispielsweise kann auch das Ventil (27) direkt zur Drucksteuerung für den Kompressor (28) eingesetzt werden.

Vom Filter (20) wird das gereinigte und im wesentlichen nur Xenon und Sauerstoff enthaltende Gas über ein Ventil (27) und einen Kompressor (28) über die Leitung (29) über drei weitere Ventile (30,31 bzw. 32) sowie zwei Leitungen (33,34) zu den Reservoiren (8 bzw. 9) zurückgeführt. Das Ventil (30) ist dabei ein Rückschlagventil, das ein Rückströmen des Gases von den Reservoiren zum Kompressor verhindert. Weiterhin ist an die Leitung (29) ein Überdruckventil (35) angeschlossen. Hinter dem Ventil (31) ist über eine Leitung (36) eine weitere Druckflasche (37) als zusätzlicher Auffangbehälter für verunreinigte Gase vorgesehen. Dieser Auffangbehälter (37) kann darüberhinaus auch dann eingesetzt werden, wenn es notwendig ist von einem Anästhesiegas, das in den Reservoiren vorhanden ist, zwischenzeitlich auf ein anderes Anästhesiegas, das von anderen Quellen beigemischt wird, überzuwechseln. Dieses andere Anästhesiemittel, das nicht zu den Reservoiren zurückgeführt werden kann, kann dann dort aufgefangen und gesammelt werden. An die Leitung (29) ist über eine Leitung (38) und ein Drosselventil (39) ein Gasanalysator (40) angeschlossen, mit dem zumindest die Xenon-Konzentration bestimmt werden kann. Vorteilhafterweise sollte dieser Gasanalysator jedoch auch die Sauerstoff-und/oder Kohlendioxyd-Konzentration messen. Das den Gasanalysator (40) durchströmende Gas wird über eine Leitung (41) wieder zur Leitung (18) zurückgeführt. Die Gasentnahme von der Leitung (29) ist bereits durch das Drosselventil (39) auf ein Minimum beschränkt. Über Ventile (42 bzw. 43) und Drosselventile (44 bzw. 45) sind die Druckflaschen (8 bzw. 9) über Leitungen (46 bzw. 47) mit weiteren Gasanalysatoren (48 bzw. 49) verbunden, mit deren Hilfe die Gaszusammensetzung in den Druckflaschen (8 bzw. 9) zumindest aber die Xenon-Konzentration in diesen Druckflaschen überwacht wird. Dass die Gasanalysatoren (48 bzw. 49) durchströmende Gas wird über Leitungen (50 bzw. 51) einer Gummiblase (52) zugeführt und dort aufgefangen. Über ein Ventil (53) kann es zu einem geeigneten Zeitpunkten zu der Leitung (18) zurückgeführt werden. Die gestrichelten Verbindungen zwischen den Druckflaschen und der Steuerelektronik sollen andeuten, dass auch noch andere Parameter, wie z.B. der Druck in den Flaschen, bestimmt werden kann.

An die Leitung (18) ist zusätzlich über ein weiteres Ventil (54) ein Douglas-Sack (55) angeschlossen. Neben den Ausgangssignalen der Lagesensoren (22 bis 24) sind die Ausgangssignale der Gasanalysatoren (13,14 sowie 40,48 und 49) auf die Steuerelektronik gegeben, wie gestrichelt angedeutet. Ebenso ist das Ausgangssignal des Unterdrucksensors (26) auf die Steuerelektronik gegeben. Mit Hilfe der Steuerelektronik werden sämtliche Ventile, der Kompressor (28) sowie der Mischer (3) und der Ventilator (1) angesteuert. Weiterhin werden die Druckflaschen (8 bzw. 9) über die Steuerelektronik angesteuert.

Die Arbeitsweise dieses Inhalations-Anästhesiegerätes ist folgende:
Über die Steuerelektronik (25) wird die gewünschte Narkosegasmischung eingestellt. Je nachdem, welche Narkosegaskonzentration gewünscht ist, wird entweder die Druckflasche (8) oder die Druckflasche (9) und möglicherweise zusätzlich die Druckflasche (4) für reinen Sauerstoff an den Mischer (3) angeschlossen. Es sei an dieser Stelle erwähnt, dass auf den Mischer (3) u.U. verzichtet werden kann. So ist es ebenso möglich, nur die Druckflaschen mit unterschiedlicher Narkosegasmischung alternativ oder zusammen an die Leitung (2) zum Ventilator (1) anzuschliessen und möglicherweise direkt an die Leitung (2) die zusätzliche Sauerstoffzufuhr. Über die Inspirationsleitung (12) wird das Gasgemisch dem Patienten zugeführt. Dabei kann sowohl während Inspirations- als auch während der Exspirations-Phase die Konzentration beispielsweise von Xenon, wenn dieses als Anästhetikum verwendet wird, und/oder $CO_2$ bestimmt werden. Über ein Sterilfilter wird das Ausatmungsgas dem Filter (20) zugeführt und anschliessend mittels des Kompressors (28) komprimiert. Der Nulldruckbehälter (21) dient als Pufferspeicher. Nur wenn, wie durch die Lagesensoren abgefühlt, ein bestimmtes Gasvolumen vorliegt, wird der Kompressor gestartet.

Eine andere Möglichkeit besteht darin, den Kompressor kontinuierlich zu betreiben und den Gasfluss zum Kompressor über das Ventil (27) zu regulieren.

Allein aus Sicherheitsgründen ist noch ein Unterdruckventil (26) vorgesehen. Sollte ein Unterdruck in der Leitung (18) entstehen, so deutet das auf einen Fehler im System hin. Daraufhin wird das Ventil (27) geschlossen und gleichzeitig das Ventil (54) geöffnet, so dass die Ausatmungsluft in den Douglas-Sack (55) geleitet werden kann. Dieser stellt ein ausreichend grosses Reservoir dar, so dass für eine gewisse Zeit die ausgeatmete Luft aufgefangen werden kann. Ohne die Anästhesie zu beeinflussen, kann nach der Störung im System gesucht werden. Das teure Narkosegas wird trotz

allem gesammelt und kann aus dem Sack wieder über das Filter und den Kompressor zu den Druckflaschen (8 bzw. 9) zurückgeführt werden.

Vorteilhafterweise wird die Narkose damit begonnen, dass zunächst die Atemwege des Patienten eine zeitlang mit reinem Sauerstoff gespült werden, um insbesondere Stickstoff und Stickstoffverbindungen aus den Atemwegen zu entfernen. Gleichzeitig werden dadurch die Leitungen des Systems gespült. In diesem Fall wird das komprimierte Gas über das Ventil (31) und die Leitung (36) zu der zusätzlichen Druckflasche (37) oder über das Ventil (35) in die Umgebung geleitet. Beispielsweise über den Gasanalysator (40) kann ermittelt werden, wenn sämtliches Stickstoffgas ausgespült ist. Danach kann der Patient zunächst von der Druckflasche (9) her mit Narkose-Frischgas mit hoher Anästhetikum-Konzentration, beispielsweise hoher Xenon-Konzentration, beatmet werden, um möglichst schnell eine ausreichend tiefe Narkose zu erzielen. Das über den Filter (20) gereinigte Gas wird wiederum über den Analysator (40) auf Xenon-Konzentration und möglicherweise anderer Konzentrationen überprüft. Da der Patient zunächst sehr viel Xenon aufnimmt, wird im ausgeatmeten Gas eine erhöhte Sauerstoffkonzentration vorliegen. Diese Konzentration nimmt sehr rasch ab und sinkt im stabilen Endzustand der Narkose unter den Wert der Sauerstoffkonzentration im Narkosefrischgas. Wird beispielsweise für eine bestimmte Narkosetiefe eine Frischgasmischung von 80% Xenon und 20% Sauerstoff gewählt, so wird ein Teil des Sauerstoffs vom Patienten verbraucht, so dass im Ausatmungsgas die Konzentration des Sauerstoffs unter 20% absinkt. Führt man beispielsweise diese Konzentration wieder einer Flasche zu, so steigt darin im Endzustand der Xenongebrauch leicht über 80%. Im eingeschwungenen Zustand kann der Patient ständig direkt aus diesem Reservoir beatmet werden, wobei dann jedes Mal die verbrauchte Sauerstoffmenge von einer separaten Gasquelle zugegeben wird. Das Ventil (31) wird umgeschaltet, so dass das komprimierte Gas über dieses und das Ventil (32) je nach der gemessenen Konzentration über den Leitungen (33 bzw. 34) entweder zu der Druckflasche (8) oder zu der Druckflasche (9) zurückgeführt wird.

Wenn eine bestimmte Narkosetiefe erreicht ist, sinkt in bekannter Weise der Anästhetikum-Verbrauch des Patienten rapide ab. Wird zunächst mit reinem Xenon beatmet, d.h. enthält ein Reservoir reines Xenon, so kann nach Erreichen der vorgesehenen Narkosetiefe über die Steuerelektronik beispielsweise auf die andere Druckflache (8) mit einer Narkosefrischgasmischung mit einer geringeren Anästhetikum-Konzentration umgeschaltet werden. Für die weitere Narkose ist es dann ausreichend, eine wesentlich geringere Menge Anästhetikum nachzuführen. Für diese Phase kann dann über die Steuerelektronik beispielsweise auf die Druckflasche (8) mit einer Narkose-Frischgasmischung mit geringerer Anästhetikum-Konzentration umgeschaltet werden. Über die Gasanalysatoren (48 und 49) wird ständig die Gaszusammensetzung in den Druckflaschen (8 bzw. 9.) überprüft. Die entsprechenden Werte können auf die Steuerelektronik (25) gegeben werden. Aus diesen Werten zusammen mit dem des zugeführten Narkose-Frischgasvolumen und beispielsweise aus dem Xenon-Gehalt des ausgeatmeten Gases, das über den Gasanalysator (14) bestimmt wird, lässt sich exakt der Xenon-Verbrauch des Patienten berechnen. Dieser Verbrauch ist ein hilfreicher Kontrollparameter für die Bestimmung der Narkosetiefe.

In diesem Ausführungsbeispiel sind lediglich zwei Druckflaschen (8 bzw. 9) für unterschiedliche Gasmischungen dargestellt. Im Rahmen der Erfindung ist es möglich, mehr als zwei Druckflaschen zu verwenden. Ebenso ist es möglich, anstelle eines Filters (20) parallel in bekannter Weise zwei derartige Filter anzuordnen, die wechselweise betrieben werden können, so dass stets ein Filter aktiv zur Reinigung des ausgeatmeten Gases benutzt wird, während das andere Filter wieder gereinigt wird.

Als Steuerelektronik kann beispielsweise ein Mikroprozessor verwendet werden.

## Patentansprüche

1. Inhalations-Anästhesiegerät mit mindestens einem Reservoir (8,9) für ein Anästhetikum sowie einem Ventilator (1), dem über eine Leitung (2) das Gas aus dem Reservoir (8,9) zugeführt ist und der in bekannter Weise über eine Inspirations- und eine Exspirationsleitung (12,17) mit den Atemwegen eines Patienten in Verbindung bringbar ist, **dadurch gekennzeichnet,** daß als Reservoir (8,9) eine Druckflasche dient, die mindestens die für die Narkose eines Patienten benötigte Menge an Anästhetikum in Gasform enthält, und daß das Ausatmungsgas über mindestens ein Filter (20), in dem vorbestimmte Gaskomponenten herausgefiltert werden, zu dem Reservoir (8,9) zurückgeführt ist.

2. Anästhesiegerät nach Anspruch 1, **dadurch gekennzeichnet,** daß zwischen dem Reservoir (8,9) und dem Ventilator (1) ein Mischer (3) angeordnet ist, dem zusätzlich mindestens ein Gas, vorzugsweise Sauerstoff, zuführbar ist.

3. Anästhesiegerät nach einem der Ansprüche 1 oder 2, **dadurch gekennzeichnet,** daß das Reservoir (8,9) ein Gasgemisch aus Anästhetikum und einem anderen Gas, vorzugsweise Sauerstoff, enthält.

4. Anästhesiegerät nach Anspruch 3, **dadurch gekennzeichnet,** daß mindestens zwei Reservoire (8,9) mit unterschiedlichen Gasmischungen und/oder mit unterschiedlichen Anästhetikum-Konzentrationen vorgesehen sind und daß an eine Leitung zwischen das Filter (20) und den Reservoiren (8,9) ein erster Gasanalysator (40) zum Messen der Gaszusammensetzung und/oder der Konzentration mindestens einer Gaskomponente angeschlossen ist und in der Leitung mindestens ein Ventil (32) angeordnet ist, das in Abhängigkeit von der gemessenen Gaszusammensetzung und/oder Gaskonzentration das Filter (20) mit dem entsprechenden Reservoir (8,9) verbindet.

5. Anästhesiegerät nach Anspruch 4, **dadurch gekennzeichnet,** daß die Anästhetikum-Konzentration in einem Reservoir (8,9), der für die gewünschte Narkosetiefe erforderlichen Konzentration entspricht.

6. Anästhesiegerät nach Anspruch 5, **dadurch gekennzeichnet,** daß die Konzentration in einem Reservoir größer und in dem anderen Reservoir kleiner als 80% ist.

7. Anästhesiegerät nach einem der Ansprüche 1 bis 6, **dadurch gekennzeichnet,** daß in dem Reservoir bzw. den Reservoiren (8,9) ein Überdruck herrscht und in der Leitung (29) zwischen Filter (20) und Reservoir (8,9) ein Kompressor (28) vorgesehen ist.

8. Anästhesiegerät nach einem der Ansprüche 1 bis 7, **dadurch gekennzeichnet,** daß an mindestens ein Reservoir (8,9) ein weiterer Gasanalysator (48,49) anschließbar ist.

9. Anästhesiegerät nach einem der Ansprüche 1 bis 8, wobei das Ausatmungsgas über eine Leitung (18) zum Filter (20) geführt ist, **dadurch gekennzeichnet,** daß an diese Leitung ein Nulldruck-Behälter (21) anschließbar ist.

10. Anästhesiegerät nach Anspruch 9, **dadurch gekennzeichnet,** daß dieser Nulldruck-Behälter (21) aus einem Balg mit Lagesensoren (22-24) besteht.

11. Anästhesiegerät nach einem der Ansprüche 1 bis 10, **dadurch gekennzeichnet,** daß zwischen den Kompressor (28) und das Reservoir bzw. die Reservoire (8,9) ein Überdruckventil (35) an der Leitung (29) angeschlossen ist.

12. Anästhesiegerät nach einem der Ansprüche 1 bis 11, **dadurch gekennzeichnet,** daß an die Leitung (29) über ein weiteres Ventil (31) ein zusätzlicher Auffangbehälter (37) für vom Filter (20) kommende Gase anschließbar ist.

13. Anästhesiegerät nach einem der Ansprüche 1 bis 12, **dadurch gekennzeichnet,** daß als Anästhetikum Xenon verwendet wird.

14. Anästhesiegerät nach einem der Ansprüche 1 bis 13, **dadurch gekennzeichnet,** daß das Filter (20) zumindest teilweise aus einem Zeoliten besteht.

15. Anästhesiegerät nach einem der Ansprüche 1 bis 14, **dadurch gekennzeichnet,** daß eine elektronische Steuereinrichtung (25) vorgesehen ist, der zumindest Signale der Gasanalysatoren (13,14,40,48,49) entsprechend den gemessenen Gasmischungen und/oder Konzentrationen zugeführt werden und die in Abhängigkeit davon zumindest das Ventil (32) ansteuert, das das Filter (20) mit den verschiedenen Reservoiren (8,9) verbindet.

**Claims**

1. Inhalation anaesthesia unit with at least one reservoir (8, 9) for an anaesthetic agent and with a ventilator (1) to which the gas is conveyed from the reservoir (8, 9) via a line (2) and which in a known manner can be brought into communication with the airways of a patient via an inspiration and an expiration line (12, 17), characterised in that the reservoir (8, 9) is a pressure tank which contains at least that amount of anaesthetic agent (in gaseous form) required for anaesthetising a patient, and in that the expiratory gas is conveyed back to the reservoir (8, 9) via at least one filter (20) in which predetermined gas components are filtered out.

2. Anaesthesia unit according to Claim 1, characterised in that a mixer (3) is arranged between the reservoir (8, 9) and the ventilator (1), to which mixer (3) it is possible to convey in addition at least one gas, preferably oxygen.

3. Anaesthesia unit according to one of Claims 1 or 2, characterised in that the reservoir (8, 9) contains a gas mixture of anaesthetic agent and another gas, preferably oxygen.

4. Anaesthesia unit according to Claim 3, characterised in that at least two reservoirs (8, 9) are provided with different gas mixtures and/or with different concentrations of anaesthetic agent, and in that a first gas analyser (40) for measuring the gas composition and/or the concentration of at least one gas component is connected to a line between the filter (20) and the reservoirs (8, 9), and there is arranged in the line at least one valve (32) which connects the filter (20) to the corresponding reservoir (8, 9) as a function of the measured gas composition and/or gas concentration.

5. Anaesthesia unit according to Claim 4, characterised in that the concentration of anaesthetic agent in one reservoir (8, 9) corresponds to the concentration required for the desired depth of anaesthesia.

6. Anaesthesia unit according to Claim 5, characterised in that the concentration in one reservoir is greater than 80% and in the other reservoir less than 80%.

7. Anaesthesia unit according to one of Claims 1 to 6, characterised in that there is an overpressure in the reservoir or reservoirs (8, 9), and a compressor (28) is provided in the line (29) between filter (20) and reservoir (8, 9).

8. Anaesthesia unit according to one of Claims 1 to 7, characterised in that a further gas analyser (48, 49) can be connected to at least one reservoir (8, 9).

9. Anaesthesia unit according to one of Claims 1 to 8, where the expiratory gas is conveyed to the filter (20) via a line (18), characterised in that a zero pressure container (21) can be connected to this line.

10. Anaesthesia unit according to Claim 9, characterised in that this zero pressure container (21) consists of a bellows with position sensors (22-24).

11. Anaesthesia unit according to one of Claims 1 to 10, characterised in that a pressure control valve (35) is connected to the line (29) between the compressor (28) and the reservoir or reservoirs (8, 9).

12. Anaesthesia unit according to one of Claims 1 to 11, characterised in that an additional collecting container (37) for gases coming from the filter (20) can be connected to the line (29) via a further valve (31).

13. Anaesthesia unit according to one of Claims 1 to 12, characterised in that the anaesthetic agent used is xenon.

14. Anaesthesia unit according to one of Claims 1 to 13, characterised in that the filter (20) consists at least partially of a zeolite.

15. Anaesthesia unit according to one of Claims 1 to 14, characterised in that an electronic control facility (25) is provided, to which at least signals from the gas analysers (13, 14, 40, 48, 49) corresponding to the measured gas mixtures and/or concentrations are conveyed, and which, as a function thereof, controls at least the valve (32) connecting the filter (20) to the different reservoirs (8, 9).

**Revendications**

1. Respirateur pour anesthésie comportant au moins un réservoir (8,9) pour un anesthésique ainsi qu'un ventilateur (1), auquel le gaz est envoyé à partir du réservoir (8,9) par l'intermédiaire d'une canalisation (2) et qui peut être raccordé, de façon connue, aux voies respiratoires d'un patient par l'intermédiaire d'un conduit d'inspiration et d'un conduit d'expiration (12,17), caractérisé par le fait qu'on utilise comme réservoir (8,9) un flacon sous pression, qui contient au moins la quantité d'anesthésique sous forme de gaz, nécessaire pour anesthésier un patient, et que le gaz expiré est envoyé au réservoir (8,9) par l'intermédiaire d'au moins un filtre (20), dans lequel des constituants prédéterminés du gaz sont séparés par filtrage.

2. Appareil d'anesthésie selon la revendication 1, caractérisé par le fait qu'entre le réservoir (8,9) et le ventilateur (1) est disposé un mélangeur (3), auquel peut être envoyé en outre au moins un gaz, de préférence de l'oxygène.

3. Appareil d'anesthésie suivant l'une des revendications 1 ou 2, caractérisé par le fait que le réservoir (8,9) contient un mélange gazeux formé par l'anesthésique et un autre gaz, de préférence de l'oxygène.

4. Appareil d'anesthésie suivant la revendication 3, caractérisé par le fait qu'il est prévu au moins deux réservoirs (8,9) possédant des mélanges gazeux différents et/ou des concentrations différentes de l'anesthésique, et qu'à une canalisation située entre le filtre (20) et le réservoir (8,9) est raccordé un premier analyseur de gaz (40) servant à mesurer la composition du gaz et/ou la concentration d'au moins un constituant du gaz, et que dans la canalisation, est installée au moins une soupape (32) qui, en fonction de la composition du gaz et/ou de la concentration du gaz mesurée, raccorde le filtre (20) au réservoir correspondant (8,9).

5. Appareil d'anesthésie selon la revendication 4, caractérisé en ce que la concentration de l'anesthésique dans un réservoir (8,9) correspond à la concentration nécessaire pour le degré d'anesthésie désiré.

6. Appareil d'anesthésie suivant la revendication 5, caractérisé par le fait que la concentration dans un réservoir est supérieure et la concentration dans l'autre réservoir est inférieure à 80 %.

7. Appareil d'anesthésie suivant l'une des revendications 1 à 6, caractérisé par le fait qu'une surpression règne dans le ou les réservoirs (9) et qu'un compresseur (28) est prévu dans la canalisation (29) située entre le filtre (20) et le réservoir (8,9).

8. Appareil d'anesthésie suivant l'une des revendications 1 à 7, caractérisé par le fait qu'un autre analyseur de gaz (48,49) peut être raccordé à au moins un réservoir (8,9).

9. Appareil d'anesthésie suivant l'une des revendications 1 à 8, dans lequel le gaz expiré est envoyé au filtre (20) par l'intermédiaire d'une canalisation (18), caractérisé par le fait qu'un récipient à pression nulle (20) peut être raccordé à cette canalisation.

10. Appareil d'anesthésie suivant la revendication 9, caractérisé par le fait que ce récipient à pression nulle (21) est constitué par un soufflet comportant des capteurs de position (22-24).

11. Appareil d'anesthésie suivant l'une des revendications 1 à 10, caractérisé par le fait qu'une soupape de sécurité (35) est raccordée à la canalisation (29) entre le compresseur (28) et le ou les réservoirs (8,9).

12. Appareil d'anesthésie suivant l'une des revendications 1 à 11, caractérisé par le fait qu'à la canalisation (29) peut être raccordé, par l'intermédiaire d'une autre soupape (31), un récipient supplémentaire de collecte (37) pour les gaz arrivant du filtre (20).

13. Appareil d'anesthésie suivant l'une des revendications 1 à 12, caractérisé par le fait qu'on utilise du xénon comme anesthésique.

14. Appareil d'anesthésie suivant l'une des revendications 1 à 13, caractérisé par le fait que le filtre (20) est constitué au moins en partie par un zéolithe.

15. Appareil d'anesthésie suivant l'une des revendications 1 à 14, caractérisé par le fait qu'il est prévu un dispositif de commande électronique (25) auquel au moins des signaux des analyseurs de gaz (13,14,40,48,49) sont envoyés en fonction des mélanges gazeux mesurés et/ou qui, en fonction de cela, commandent au moins la soupape (32) qui relie le filtre (20) aux différents réservoirs (8,9).